Europäisches Patentamt

European Patent Office

Office européen des brevets

(19)

(11) Veröffentlichungsnummer : **0 457 123 B1**

# (12) EUROPÄISCHE PATENTSCHRIFT

(45) Veröffentlichungstag der Patentschrift :
**19.07.95 Patentblatt 95/29**

(51) Int. Cl.$^6$ : **G01N 33/543,** G01N 33/545,
G01N 33/58, G01N 33/94

(21) Anmeldenummer : **91107169.4**

(22) Anmeldetag : **03.05.91**

(54) **Testträger für die analytische Bestimmung eines Bestandteils einer Probenflüssigkeit.**

(30) Priorität : **14.05.90 DE 4015378**

(43) Veröffentlichungstag der Anmeldung :
**21.11.91 Patentblatt 91/47**

(45) Bekanntmachung des Hinweises auf die
Patenterteilung :
**19.07.95 Patentblatt 95/29**

(84) Benannte Vertragsstaaten :
**AT BE CH DE DK ES FR GB GR IT LI LU NL SE**

(56) Entgegenhaltungen :
**EP-A- 0 274 911**
**EP-A- 0 318 777**

(73) Patentinhaber : **BOEHRINGER MANNHEIM GMBH**
**D-68298 Mannheim (DE)**

(72) Erfinder : **Wilk, Hans-Erich, Dr.**
**Theodor Heuss-Strasse 2**
**W-6141 Einhausen (DE)**
Erfinder : **Schneider, Erich, Dr.**
**Märker Querschlag 6**
**W-6800 Mannheim 31 (DE)**
Erfinder : **Marschall, Andreas, Dr.**
**Levkojenweg 18**
**W-6800 Mannheim 31 (DE)**
Erfinder : **Bleisteiner, Manfred**
**Wallstadter Strasse 46**
**W-6800 Mannheim 51 (DE)**

(74) Vertreter : **Pfeifer, Hans-Peter, Dr.rer.nat.**
**Patentanwalt**
**Nowackanlage 15**
**D-76137 Karlsruhe (DE)**

## Beschreibung

Die Erfindung betrifft einen Testträger für die analytische Bestimmung eines Bestandteils einer Probenflüssigkeit mit Hilfe einer spezifischen Bindungsreaktion zweier bioaffiner Bindungspartner mit einer Mehrzahl von Testschichten, die so angeordnet sind, daß sie von der Flüssigkeit nacheinander benetzt werden und einen Flüssigkeitstransportweg bilden.

Qualitative und quantitative analytische Bestimmungen werden vielfach mit Hilfe von Testträgern durchgeführt. Dabei sind kapillaraktive Testschichten, welche üblicherweise aus saugfähigen Schichtmaterialien wie Papieren, Vliesen oder porösen Kunststoffen bestehen, in verschiedener Anordnung auf oder an einem Basiselement derart befestigt, daß sie nacheinander von der Flüssigkeit durchströmt werden. Die Testschichten enthalten Reagenzien, die man insgesamt als Reagenzsystem bezeichnet und deren Reaktion mit der Probe zu einem Nachweissignal in Form einer physikalisch nachweisbaren Veränderung, insbesondere einer Farbänderung in einer Nachweisschicht führt.

Testträger sind in verschiedenen äußeren Gestaltungen bekannt. Von praktischer Bedeutung sind insbesondere streifenförmige Testträger, bei denen ein Kunststoffstreifen das Basiselement bildet, auf dem die Testschichten befestigt sind, sowie Testträger in Form quadratischer Plättchen, bei denen die Testschichten in einem Rahmen eingerahmt sind. In der angelsächsischen Literatur werden sie als "test carriers", vielfach auch als "solid state analysis elements" bezeichnet.

Auch für Testverfahren, die auf einer spezifischen Bindungsreaktion zweier bioaffiner Bindungspartner beruhen, sind schon zahlreiche Testträger vorgeschlagen worden. Spezifische Bindungsreaktionen in diesem Sinne sind insbesondere immunologische Interaktionen, also Wechselwirkungen zwischen Antigenen bzw. Haptenen einerseits und Antikörpern andererseits. Es können jedoch auch andere spezifische bioaffine Wechselwirkungen verwendet werden, wie Biotin-Streptavidin, Lektin-Zucker oder eine Wirkstoff-Rezeptor-Wechselwirkung. Im folgenden wird ohne Beschränkung der Allgemeinheit beispielhaft auf immunologische Wechselwirkungen Bezug genommen.

Bei solchen Tests stellt sich -unabhängig von dem Verfahrensgang im einzelnen- vielfach die Aufgabe, den bei einer Bindungsreaktion gebundenen Anteil von dem nichtgebundenen Anteil zu trennen (bound/free-Trennung, B/F-Trennung). Dies erfolgt bei konventionellen immunologischen Tests durch aufwendige Wasch- und Absaugschritte. Bei trägergebundenen Tests wird vielfach eine Fixierungsschicht für die Trennung verwendet, die eine poröse Trägermatrix aufweist, an der einer der beiden Bindungspartner der spezifischen Bindungsreaktion fixiert ist. Die Fixierungsschicht ist auf dem Testträger so angeordnet, daß sie von der Flüssigkeit, welche den zweiten Bindungspartner enthält, durchströmt wird. Beim Durchströmen findet die Bindungsreaktion statt, d.h. ein Teil des zweiten Bindungspartners wird an den trägerfixierten ersten Bindungspartner aufgrund der spezifischen Bindungsreaktion gebunden und damit zugleich fixiert. Der nicht gebundene Teil des zweiten Bindungspartners bleibt frei beweglich und strömt weiter durch die Fixierungsschicht in mindestens eine im Flüssigkeitstransportweg hinter der Fixierungsschicht angeordnete saugfähige Schicht.

Diese Art der Durchführung spezifischer Bindungsreaktionen einschließlich B/F-Trennung auf Testträgern erscheint einfach. Ihre Realisierung erweist sich jedoch als außerordentlich anspruchsvoll. Ein erstes Problem ist die lange Reaktionszeit spezifischer Bindungsreaktionen. Obwohl die früher erforderlichen Reaktionszeiten von vielen Stunden durch die Entwicklung besonders reaktionsschneller Bindungspartner erheblich reduziert wurden, sind sie immer noch lang im Verhältnis zu der Zeit, die die Probenflüssigkeit benötigt, um eine Testschicht zu durchqueren.

Für immunologische Analysen wurden deswegen Testträger entwickelt, bei denen die Probenflüssigkeit die Fixierungsschicht nicht senkrecht zu ihrer Oberfläche durchquert, sondern parallel zur Oberfläche der Fixierungsschicht, also in deren Längsrichtung, strömt. Ähnlich einem Chromatographieverfahren steht dadurch ein verhältnismäßig langer Weg (üblicherweise mehrere Zentimeter) für den Ablauf der Bindungsreaktion und die B/F-Trennung zur Verfügung. Derartige Testträger sind in den US-Patenten 4 361 537 und 4 861 711 beschrieben.

Diese Konstruktion ist jedoch aufwendig in der Herstellung. Die Handhabung wird durch die Größe der Testträger beeinträchtigt. Außerdem führt der lange Kontakt der Probenflüssigkeit mit der Fixierungsschicht dazu, daß eine teilweise Rückreaktion eintritt, bei der ein Teil des bereits gebundenen zweiten Bindungspartners sich von den Bindungsstellen wieder löst.

Insofern ist eine Testträgerkonstruktion konzeptionell überlegen, bei der die Fixierungsschicht auf dem Testträger so angeordnet ist, daß sie senkrecht zu ihrer Oberfläche von der Flüssigkeit mit dem zweiten Bindungspartner durchströmt wird. Ein solcher Testträger ist beispielsweise in der EP-A-318 777 beschrieben. Dabei müssen allerdings extreme Anforderungen erfüllt werden. Insbesondere ist die Reaktionszeit für die immunologische Bindungsreaktion extrem kurz. Sie liegt typischerweise unter 10 sec. Dennoch soll, um eine ausreichende Genauigkeit zu erreichen, eine möglichst vollständige Bindung (mehr als 90 %) erreicht werden. Diese Anforderungen werden noch zusätzlich dadurch erhöht, daß bei modernen Analysetestträgern mit extrem kleinen Probenmengen (typisch weniger als 30µl) gearbeitet wird und deswegen die Fixierungsschicht extrem dünn (typische Dicke kleiner als 0,5 mm) ausgebildet ist.

Der Erfindung liegt daher die Aufgabe zugrunde, einen weitgehend vollständigen Ablauf der Bindungsreaktion und Abtrennung des gebundenen Anteils in kürzester Zeit, nämlich beim Durchströmen einer Fixierungsschicht senkrecht zu ihrer Oberfläche zu ermöglichen.

Die Aufgabe wird bei einem Testträger der vorstehend definierten Art dadurch gelöst, daß die Trägermatrix der Fixierungsschicht eine mikroporöse Kunststoffschicht mit einer Porengröße P von mindestens 0,01µm ist, und die Größe D des zweiten Bindungspartners und die Porengröße P der Kunststoffschicht so aufeinander abgestimmt sind, daß die Porengröße P mindestens zwei und höchstens zehn mal so groß wie die Größe D des zweiten Bindungspartners ist.

Die Verwendung poröser Kunststoffschichten als Trägermatrix für immunologische Reagenzbestandteile ist bekannt. Sie wird beispielsweise in der DE-A-33 29 728 erwähnt, in der zahlreiche verschiedene Materialien gegenübergestellt werden. Speziell mit porösen Kunststoffmembranen als Trägermaterial für immunologische Tests befassen sich die EP-A-194 578 und die EP-A-274 911. Auf die speziellen Erfordernisse einer dünnen, senkrecht zu ihrer Oberfläche durchströmten Fixierungsschicht wird dort jedoch nicht eingegangen.

Erfindungsgemäß wird eine weitgehend vollständige Bindungsreaktion in außerordentlich kurzer Zeit beim Durchströmen einer extrem dünnen Fixierungsschicht (Dicke vorzugsweise kleiner als 0,4 mm, besonders bevorzugt kleiner als 0,25 mm) erreicht. Dies ist auf die Kombination der beanspruchten Maßnahmen zurückzuführen:

- Einerseits soll ein <u>Minimalwert</u> der Porengröße P eingehalten werden.
- Andererseits wird mindestens eine der Größen (Porengröße P und Größe D des zweiten Bindungspartners) in Relation zu der anderen Größe so abgestimmt, daß die genannten Grenzwerte eingehalten werden.

Die zweite Bedingung wird gemäß einer bevorzugten Ausführungsform dadurch realisiert, daß der zweite Bindungspartner durch chemische Bindung auf ein im Vergleich zum Molekulargewicht seiner monomeren Form erhöhtes Molekulargewicht vergrößert wird. Überraschenderweise führt diese Vergrößerung zu einer erheblich schnelleren und vollständigeren Bindung, obwohl die Gesamtzahl der Bindungsstellen nicht erhöht wird.

Die Vergrößerung des zweiten Bindungspartners kann in der Weise realisiert werden, daß ein Oligomer oder Polymer des zweiten Bindungspartners verwendet wird. Aggregate entstehen bei der Herstellung solcher Reagenzien vielfach von selbst. Beispielsweise haben Konjugate aus Antikörper und Enzym (AkE) meist aktive Gruppen im Bereich der Brücke zwischen Enzym und Antikörper. Bisher wurden, beispielsweise mit einem Molekularsieb, aus dem so entstandenen Gemisch möglichst kleine Fraktionen ausgewählt. Ziel war es, sogenannte 1:1-Konjugate zu verwenden, bei denen jeweils ein Antikörper mit einem Enzymmolekül konjugiert ist. Teilweise wurde sogar das Enzym durch Verwendung von FAB-Fragmenten noch zusätzlich verkleinert, um eine möglichst hohe Empfindlichkeit zu erreichen.

Andere Möglichkeiten der Vergrößerung des zweiten Bindungspartners sind die Verwendung eines polymeren Markierungsenzyms (beispielsweise bei der Herstellung von Antikörper-Enzym-Konjugaten) oder die Bindung an hinsichtlich der bioaffinen Wechselwirkung inerte Teilchen, beispielsweise Makromoleküle oder Latex-Partikel.

Überraschenderweise hat sich im Rahmen der vorliegenden Erfindung gezeigt, daß die mit der Größenzunahme des zweiten Bindungspartners verbundene verlangsamte Diffusion nicht zu einer Erhöhung der Reaktionszeit der Bindungsreaktion führt.

Es werden zahlreiche mikroporöse Kunststoff-Schichtmaterialien mit einer sehr gleichmäßigen Porengrößenverteilung angeboten. Ihre Porengröße wird üblicherweise vom Hersteller angegeben. Für die Erfindung sind insbesondere Polyvinyldiflorid, Polyamid, Polystyrol und Nitrocellulose als Schichtmaterialien geeignet.

Der zweite Bindungspartner liegt notwendigerweise als Gemisch verschiedener Teilchengrößen vor. Die Angabe der Teilchengröße D bezieht sich auf den Mittelwert dieses Gemisches. Die Teilchengröße in diesem Sinne läßt sich durch Filtrationsversuche bestimmen, wobei eine Flüssigkeit mit einer Teilchengröße D im Sinne der Erfindung dadurch charakterisiert ist, daß von einer mikroporösen Kunststoffschicht mit einer Porengröße P = 1,5 D maximal 10 % der Teilchen zurückgehalten werden, während von einer mikroporösen Kunststoffschicht mit einer Porengröße P = 0,67 D mindestens 90 % der Teilchen zurückgehalten werden.

Die Bindung des ersten Bindungspartners an die mikroporöse Kunststoffschicht kann nach den üblichen Verfahren erfolgen. Dabei werden üblicherweise durch Anätzen reaktive organische Gruppen an der Kunststoffoberfläche erzeugt, an die beispielsweise Antigene und Antikörper kovalent gebunden werden können. Derartige Verfahren werden beispielsweise in den eingangs genannten Literaturstellen beschrieben, auf die hier insoweit Bezug genommen wird.

Die Erfindung wird im folgenden anhand von in den Figuren schematisch dargestellten Ausführungsbeispielen näher erläutert. Es zeigen:

Fig. 1    einen Querschnitt durch die Schichtenfolge eines mehrschichtigen Testträgers,

Fig. 2    eine alternative Ausführungsform eines Testträgers in perspektivischer Darstellung.

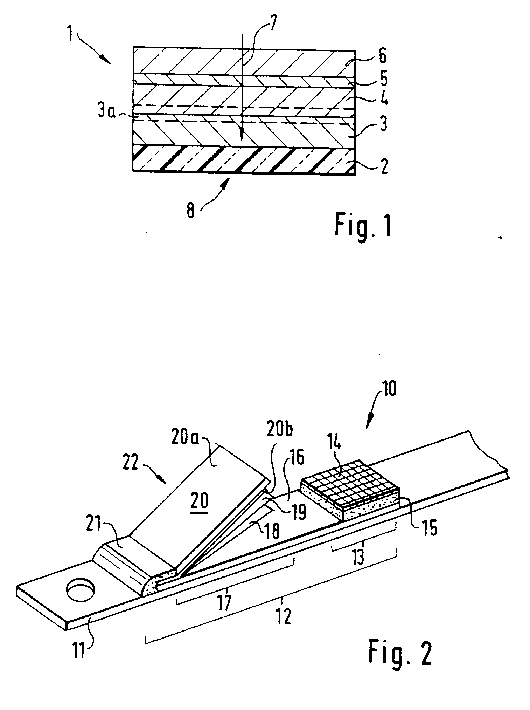

Die in Fig. 1 dargestellte Schichtenfolge eines erfindungsgemäßen Testträgers 1 besteht aus einer Basisschicht 2 aus einem durchsichtigen Kunststoffmaterial, einer Farbnachweisschicht 3, einer Fixierungsschicht 4, einer Strömungskontrollschicht 5 und einer Konjugatschicht 6. Die Schichten 3-6 sind auf der Basisschicht 2 derartig befestigt, daß sie in vollflächigem Kontakt zueinander stehen, der einen gleichmäßigen Flüssigkeitsübertritt in Richtung quer zur Schichtenoberfläche ermöglicht. Die dadurch gebildete Flüssigkeitstransportstrecke ist mit dem Pfeil 7 bezeichnet.

Die Funktion des beispielhaft dargestellten Testträgers wird anhand eines Testablaufs erläutert, der dem immunenzymometrischen Testprinzip nachgebildet ist. Soll beispielsweise ein in der Probe enthaltenes Antigen (Ag) bestimmt werden, so läuft die Analyse folgendermaßen ab.

Ein Tropfen Probe, beispielsweise Blut, wird auf die Konjugatschicht 6 aufgegeben und breitet sich darauf aus. Die Schicht 6 dient dabei als Flüssigkeitszufuhrschicht, für die auf der Flüssigkeitstransportstrecke 7 nachfolgenden Schichten.

In der Konjugatschicht 6 ist ein enzymatisch markierter Antikörper (Ak) für das Ag im Überschuß gegenüber der maximalen Ag-Konzentration in der Probe enthalten. Das Antikörper-Enzym-Konjugat (AkE) wird von der eindringenden Probenflüssigkeit gelöst. Dabei bilden sich Komplexe zwischen dem AkE und dem Ag, die mit Ag-AkE bezeichnet werden. Da das AkE im Überschuß vorhanden ist, bleibt bei Einstellung des Gleichgewichts freies AkE übrig.

Die Strömungskontrollschicht 5 dient dazu, den Zeitpunkt des Flüssigkeitsübertritts von der Konjugatschicht 6 in die Fixierungsschicht 4 zu kontrollieren. Sie kann insbesondere als verzögert lösliche Sperrschicht ausgebildet sein, wobei sie aus einem von der Flüssigkeit nur langsam aufgelösten schichtbildenden Material besteht. Diese Verfahrensweise ist bekannt und muß hier nicht näher erläutert werden. Durch die Strömungskontrollschicht 5 läßt sich erreichen, daß die Komplexbildung zwischen Ag und AkE in der Konjugatschicht 6 praktisch abgeschlossen ist, bevor die Flüssigkeit in die Fixierungsschicht 4 eindringt. Es soll jedoch betont werden, daß die Strömungskontrollschicht optional ist.

Die Fixierungsschicht 4 dient dazu, das den weiteren Nachweis störende AkE durch immunologische Bindung zu beseitigen. Sie enthält das Ag oder ein Analogon hierzu (eine andere mit dem Antikörper spezifisch bindungsfähige Substanz) in trägerfixierter Form. Beim Durchströmen der Flüssigkeit durch die Fixierungsschicht 4 bindet das nichtkomplexierte AkE mit dem trägerfixierten Ag(f), während die Ag-AkE Komplexe ungehindert weiterströmen können. Dadurch wird die gewünschte Abtrennung erreicht.

Die enzymatisch markierten Komplexe dringen in die Nachweisschicht 3 ein, welche ein farbbildendes Substrat S für das Markierungsenzym E enthält. Durch die Reaktion zwischen Enzym und Substrat ändert sich dessen Farbe und die Farbbildung ist ein Nachweis für die Konzentration des Analyten Ag.

Da das Substrat in der Nachweisschicht 3 im Regelfall löslich ist, besteht das Risiko, daß es aus der Schicht 3 in die Schicht 4 und möglicherweise sogar in die Schichten 5 und 6 diffundiert, nachdem es von der Probe gelöst worden ist. Dabei kommt es mit Markierungsenzym E in Kontakt, welches nicht für die Analyse charakteristisch ist. Dies kann zu einer Verfälschung des Meßergebnisses führen, wenn die Farbbildung von der Auswertungsseite 8 her wahrnehmbar ist. Um dies zu verhindern, ist die Farbnachweisschicht 3, beispielsweise durch Einlagerung von $TiO_2$, optisch undurchlässig gestaltet. Es kann auch zwischen den Schichten 3 und 4 eine spezielle flüssigkeitsdurchlässige strahlungsblockierende Schicht 3a vorgesehen sein, die in der Figur gestrichelt angedeutet ist.

Das geschilderte Testprinzip ist bekannt und muß deshalb nicht weiter erläutert werden. Es ist für den erfindungsgemäßen Testträger bevorzugt, wobei selbstverständlich in analoger Weise ein in der Probe vorhandener Antikörper bestimmt werden kann, wenn in der Schichtenfolge des Testträgers jeweils Antikörper und Antigen gegeneinander vertauscht werden.

Die Erfindung ist jedoch nicht auf ein solches Verfahren beschränkt. Vielmehr kann sie unabhängig von Einzelheiten des Verfahrensablaufes überall vorteilhaft eingesetzt werden, wo beim Passieren einer Fixierungsschicht 4 auf sehr kurzer Strecke und in sehr kurzer Zeit eine praktisch vollständige Bindungsreaktion zwischen einem trägerfixierten Bindungspartner und einem mit der Probenflüssigkeit transportierten Bindungspartner ablaufen soll.

Figur 2 zeigt eine besonders bevorzugte Ausführungsform, mit der eine Analyse in kurzer Zeit mit hoher Genauigkeit und mit einer sehr kleinen Probenmenge möglich ist.

Der Testträger 10 hat auf einer Basisschicht 11 eine Nachweiszone 12, auf der die Testschichten angeordnet sind, die die Flüssigkeitstransportstrecke bilden.

In einer Probenaufgabezone 13 ist unter einem Abdecknetz 14 eine Erythrozytenabtrennschicht 15 vor-

EP 0 457 123 B1

gesehen. Eine zwischen dieser und der Basisschicht 11 angeordnete Konjugatschicht 16 verläuft von der Probenaufgabezone 13 in eine Auswertezone 17 parallel zur Oberfläche der Basisschicht 11. In der Auswertezone 17 ist eine Fixierungsschicht 18, eine optische Sperrschicht 19 und eine Farbnachweisschicht 20 derartig befestigt, daß sie im dargestellten Ausgangszustand des Testträgers 10 nicht in Fluidkontakt zu der als Flüssigkeitszufuhrschicht dienenden Konjugatschicht 16 stehen, jedoch durch externe Manipulation in Flüssigkeitskontakt zu dieser gebracht werden können. Im dargestellten Fall ist dies einfach dadurch realisiert, daß die Schichten 18,19 und 20 jeweils mit einer Kante mittels eines Schmelzkleberstreifens 21 derartig an der Basisschicht 11 befestigt sind, daß sie im Ausgangszustand des Testträgers klappenartig schräg nach oben von der Konjugatschicht 16 abstehen. Auch diese insgesamt mit 22 bezeichnete Konstruktion eines Strömungskontrollelements als Klappe ist bekannt und muß im einzelnen nicht näher erläutert werden.

Im Zusammenhang mit der vorliegenden Erfindung erweist sich die dargestellte Schichtenfolge der separaten Schichten 18,19 und 20 als besonders vorteilhaft. Die Fixierungsschicht 18 kann erfindungsgemäß außerordentlich dünn, vorzugsweise weniger als 0,5 mm, besonders bevorzugt sogar weniger als 0,25 mm dick sein. Dadurch nimmt sie nur wenig Probenflüssigkeit auf. Die Farbnachweisschicht 20 besteht aus einer Trägerfolie 20a und aus einer Reagenzfilmschicht 20b, die auf die in der Konjugatschicht 16 zugewandte Seite der Trägerfolie 20a beschichtet und ebenfalls außerordentlich dünn (Schichtdicke weniger als 200µm) ausgebildet ist. Dies erlaubt einen intensiven Farbnachweis mit einer geringen Probenmenge. Voraussetzung für eine ungestörte Auswertung der Farbbildung ist dabei eine vollständig lichtundurchlässige optische Sperrschicht 19. Wegen des geringen Flüssigkeitsbedarfes der Schichten 18 und 20b kann die optische Sperrschicht 19 ausreichend dick (Schichtdicke mindestens 50µm) ausgebildet werden, ohne den Gesamtbedarf an Probenflüssigkeit über den gewünschten Grenzwert (im Beispielsfall 30µl) ansteigen zu lassen. Die optische Sperrschicht kann auch unmittelbar auf die Reagenzfilmschicht 20b beschichtet sein, wodurch sich eine weitere Verminderung der Flüssigkeitsaufnahme ergibt.

Der Testablauf entspricht dem zuvor im Zusammenhang mit Fig. 1 beschriebenen, wobei die Schicht 15 dafür sorgt, daß die Erythrozyten aus dem Blut abgetrennt werden, so daß sie die nachfolgenden Reaktionsschritte (insbesondere den Farbnachweis in der Schicht 20) nicht mehr stören können.

Die dargestellte Konstruktion mit einer Klappe erlaubt eine noch zuverlässigere zeitliche Trennung der einzelnen Reaktionsschritte als bei der Ausführungsform gemäß Fig. 1. Dies ist von besonderem Vorteil in Verbindung mit der hervorragenden Trennwirkung der erfindungsgemäßen Fixierungsschicht 18, weil beispielsweise bei dem geschilderten Reaktionsablauf vor dem Andrücken der Klappe 18,19,20 gegen die Konjugatschicht 16 ein erster Reaktionsschritt vollständig und kontrolliert ablaufen kann, während die nachfolgende Abtrennreaktion mit Hilfe der Fixierungsschicht 18 schnell und dennoch vollständig abläuft. In der Praxis bedingt die Konstruktion mit einer klappenartig befestigten Fixierungsschicht allerdings, daß die Fixierungsschicht 18 in außerordentlich kurzer Zeit (typischerweise weniger als 10 sec.) durchströmt wird. Dennoch wird erfindungsgemäß eine ausreichende Trennwirkung erreicht, wie im folgenden anhand von Ausführungsbeispielen belegt wird.

Beispiel 1:

Es werden drei Muster A,B,C von Testträgern gemäß Fig. 2 hergestellt, die sich nur hinsichtlich der Porengröße P der Fixierungsschicht 18 und der Teilchengröße D des Konjugats in der Konjugatschicht 16 voneinander unterscheiden.

Die Konjugatschicht besteht aus einem Glasfaservlies, welches zur Erleichterung der Ablösung mit Polyvinylalkohol beschichtet ist. Diese Schicht wird bei Muster A mit einem Anti-Theophyllin-Antikörper-βGalaktosidase-Konjugat (Molekulargewicht M ca. 15 MDa, Größe D = ca. 0,08µm) in einer Aktivität von 150 mU/cm² getränkt. Bei Vergleichsmuster B und C wird ein niedermolekulares Konjugat (M ca. 0,7 MDa, Größe D = ca. 0,015µm) in gleicher Aktivität eingesetzt.

Die Fixierungsschicht 18 besteht bei den Mustern A und B aus einem mikroporösen Kunststoff mit einer Porengröße P = 0,2µm. Diese Trägermatrix ist kovalent beladen mit einem Theophyllin-Polyhapten (Protein belegt mit Theophyllin) mit einer Beladungsdichte von 50µg/cm². Bei einem Vergleichsmuster C wird eine in ihren übrigen Eigenschaften identische Matrix mit einer Porengröße P = 1,2µm verwendet.

Die Nachweisschicht und die optische Sperrschicht bestehen aus einem Dispersions-Reagenzfilm unter Verwendung von Propriofan (Warenzeichen der BASF AG) als Filmbildner, in den Chlorphenolrotgalaktosid als farbbildendes Substrat eingebettet ist. Die optische Sperrschicht besteht aus Titandioxid, welches unmittelbar auf die Farbnachweisschicht beschichtet ist.

Mit den drei Mustern wird Serum bzw. Blut mit insgesamt sechs verschiedenen Konzentrationen an Theophyllin untersucht, wozu das kommerziell erhältliche Gerät Reflotron (Warenzeichen der Boehringer Mannheim GmbH) eingesetzt wird. Die Klappe 20 wird dabei mit einer an diesem Gerät vorhandenen Mechanik ge-

5

gen die Flüssigkeitszufuhrschicht 16 gedrückt, wobei die Verschlußzeit 0,3 sec. beträgt. Diese Zeit ist ein ungefähres Maß für die Zeit, in der die Fixierungsschicht 18 durchströmt wird.

Beim Reaktionsablauf wird folgende zeitliche Folge eingehalten:

Vorreaktionszeit (Lösung des Konjugats und Komplexbildung mit dem Antigen in der Probe): 120 sec, danach Schließen der Klappe;

Messung der Farbbildung nach 120 sec.

Dabei ergeben sich folgende Resultate:

| Theophyllin mg/L | Muster A %Rem | Muster B %Rem | Muster C %Rem |
|---|---|---|---|
| 0 | 61,3 | 43,2 | 38,7 |
| 5 | 47,5 | 33,0 | 27,9 |
| 10 | 43,0 | 31,5 | 27,0 |
| 15 | 38,8 | 30,3 | 26,7 |
| 25 | 32,7 | 30,7 | 26,6 |
| 39 | 30,1 | 30,5 | 27,6 |

% Rem = Diffuse Reflexion, bezogen auf einen weißen Standard.

Es zeigt sich, daß mit dem erfindungsgemäßen Muster A ein Signalhub von insgesamt 30 % Rem erreicht wird, während bei den Vergleichsmustern erheblich schlechtere Werte (ca. 13 % bzw. 11 %) beobachtet werden. Der hohe Signalhub erlaubt bei gegebener Genauigkeit der optischen Auswertung eine hohe Präzision der Analyse. Bei den Vergleichsversuchen ist die Genauigkeit erheblich schlechter. Im Bereich höherer Konzentrationen der Probe ist dabei keine sinnvolle Auswertung möglich.

Beispiel 2:

Es werden zwei Muster D und E von Testträgern gemäß Fig. 2 hergestellt, welche abgesehen von den folgenden Besonderheiten dem Beispiel 1 entsprechen.

In die Konjugatschicht 16 ist zusätzlich biotinyliertes Theophyllin (Theophyllin-8-Carboxypropyl-Biotin) mit einer Konzentration von 0,5 μg/ml imprägniert. Im übrigen entspricht die Schicht 15 bei Muster D der Schicht 15 von Muster A (hochmolekulares Konjugat) und bei Muster E dem Muster B und C (niedermolekulares Konjugat) in Beispiel 1.

Die Fixierungsschicht 18 hat sowohl bei Muster D als auch bei Muster E eine Porengröße P = 0,65μm. Sie ist kovalent beladen mit Streptavidin mit einer Beladungsdichte von ca. 20 μg/cm$^2$.

Mit beiden Mustern wird Serum mit verschiedenen Konzentrationen an Theophyllin vermessen. Die Meßtechnik ist die gleiche wie bei Beispiel 1, jedoch wird die Klappe langsamer geschlossen. Durch eine Verschlußzeit von 8 sec. wird sichergestellt, daß das Streptavidin nach dem Kontakt mit der Probenflüssigkeit in ausreichendem Maße renaturiert wird.

Das Testprinzip weicht in diesem Fall von dem im Zusammenhang mit Fig. 2 und Beispiel 1 beschriebenen Testprinzip ab. In der Konjugatschicht 15 verdrängt das Ag aus der Probe das biotinylierte Antigen (B-Ag) aus seiner Bindung mit dem Antikörper-Enzym-Konjugat (AkE). Es bilden sich Komplexe B-Ag-AkE und Ag-AkE, wobei die Konzentration der letztgenannten Komplexe umso höher ist, je mehr Antigen in der Probe war.

Nach Schließen der Klappe 22 dringen beide Komplexe in die Fixierungsschicht 18 ein, wobei die biotinhaltigen Komplexe an das Streptavidin binden und dadurch festgehalten werden. Auch in diesem Fall ist die Farbbildung in der Farbnachweisschicht 20 ein Maß für die Antigenkonzentration.

Im Beispielsfall resultieren folgende Ergebnisse:

6

| Theophyllin mg/L | Muster D %Rem | Muster E %Rem |
|---|---|---|
| 0 | 55,9 | 38,3 |
| 5 | 48,8 | 35,7 |
| 10 | 43,8 | 32,5 |
| 20 | 39,5 | 30,7 |
| 30 | -- | 30,4 |
| 40 | 31,7 | -- |

Es wird deutlich, daß das erfindungsgemäße Muster D einen wesentlich höheren Signalhub als das Muster E erzeugt.

**Patentansprüche**

1. Testträger für die analytische Bestimmung eines Bestandteils einer Probenflüssigkeit mit Hilfe einer spezifischen Bindungsreaktion zweier bioaffiner Bindungspartner

   mit einer Mehrzahl von Testschichten, die so angeordnet sind, daß sie von der Flüssigkeit nacheinander benetzt werden und einen Flüssigkeitstransportweg (7) bilden,
   wobei eine der Testschichten eine Fixierungsschicht (4,18) ist, die eine poröse Trägermatrix aufweist, an der der erste der beiden Bindungspartner fixiert ist,
   wobei die Fixierungsschicht (4,18) auf dem Testträger (1,10) so angeordnet ist, daß sie von der Flüssigkeit, welche den zweiten Bindungspartner enthält, senkrecht zu ihrer Oberfläche durchströmt wird, und wobei mindestens eine saugfähige Schicht (3,19,20) im Flüssigkeitstransportweg (7) hinter der Fixierungsschicht (4,18) vorgesehen ist,
   **dadurch gekennzeichnet,** daß
   die Trägermatrix der Fixierungsschicht (4,18) eine mikroporöse Kunststoffschicht mit einer Porengröße P von mindestens 0,01μm ist, und
   die Größe D des zweiten Bindungspartners und die Porengröße P der Kunststoffschicht so aufeinander abgestimmt sind, daß die Porengröße P mindestens zwei und höchstens zehn mal so groß wie die Größe D des zweiten Bindungspartners ist.

2. Testträger nach Anspruch 1, **dadurch gekennzeichnet,**
   daß der zweite Bindungspartner durch chemische Bindung auf ein höheres Molekulargewicht vergrößert ist, als seiner monomeren Form entspricht.

3. Testträger nach Anspruch 2, **dadurch gekennzeichnet,**
   daß der zweite Bindungspartner ein Oligomer oder Polymer der monomeren Form ist.

4. Testträger nach Anspruch 2, **dadurch gekennzeichnet,**
   daß der zweite Bindungspartner durch chemische Bindung mit einem hinsichtlich der bioaffinen Bindungsreaktion inerten Bindungspartner vergrößert ist.

5. Testträger nach einem der vorhergehenden Ansprüche, **dadurch gekennzeichnet,** daß der erste Bindungspartner Streptavidin und der zweite Bindungspartner Biotin enthält.

6. Testträger nach Anspruch 1, **dadurch gekennzeichnet,**
   daß in dem Flüssigkeitstransportweg hinter der Fixierungsschicht (18) eine Farbnachweisschicht (20) und zwischen der Fixierungsschicht (18) und der Farbnachweisschicht (20) eine optische Sperrschicht (19) angeordnet ist.

7. Testträger nach einem der vorhergehenden Ansprüche, **dadurch gekennzeichnet,** daß die Dicke der Fixierungsschicht kleiner als 0,5 mm, insbesondere kleiner als 0,25 mm ist.

8. Testträger nach einem der vorhergehenden Ansprüche, **dadurch gekennzeichnet,** daß der Fixierungsschicht ein Strömungskontrollelement (5,22) zugeordnet ist, durch das der Zeitpunkt des Durchströmens der Probenflüssigkeit durch die Fixierungsschicht steuerbar ist.

9. Testträger nach Anspruch 8, **dadurch gekennzeichnet**,
daß die Fixierungsschicht (18) an dem Testträger (10) derart befestigt ist, daß sie im Ausgangszustand des Testträgers (10) nicht in Fluidkontakt zu einer in dem Flüssigkeitstransportweg vorgelagerten Flüssigkeitszufuhrschicht (16) steht, jedoch in Fluidkontakt mit der Flüssigkeitszufuhrschicht (16) bringbar ist.

10. Testträger nach Anspruch 9 in Verbindung mit Anspruch 6, **dadurch gekennzeichnet**, daß die Fixierungsschicht (18), die optische Sperrschicht (19) und die Farbnachweisschicht (20) in dieser Reihenfolge klappenartig über der Flüssigkeitszufuhrschicht angeordnet sind.

11. Testträger nach einem der vorhergehenden Ansprüche, **dadurch gekennzeichnet**, daß die Fixierungsschicht (18) aus Polyvinyldifluorid, Polyamid, Polystyrol oder Nitrocellulose besteht.

## Claims

1. Test carrier for the analytical determination of a constituent of a sample fluid by means of a specific binding reaction between two binding partners having biological affinity to each other
   with a plurality of test layers which are so disposed that they are wetted successively by the fluid and form a fluid transport path (7),
   in which one of the test layers is a fixing layer (4, 18) comprising a porous carrier matrix to which the first of the two binding partners is fixed,
   in which the fixing layer (4, 18) is disposed on the test carrier (1, 10) such that the fluid containing the second binding partner flows through it at right angles to its surface, and
   in which at least one absorbent layer (3, 19, 20) is provided in the fluid transport path (7) downstream from the fixing layer (4, 18),
   characterized in that
   the carrier matrix of the fixing layer (4, 18) is a microporous plastics layer having a pore size P of at least 0.01 $\mu$m, and
   the size D of the second binding partner and the pore size P of the plastics layer are adjusted to one another such that the pore size P is at least twice and at most ten times as great as the size D of the second binding partner.

2. Test carrier according to claim 1, characterized in that the size of the second binding partner is increased by chemical bonding to a higher molecular weight than the weight which corresponds to its monomeric form.

3. Test carrier according to claim 2, characterized in that the second binding partner is an oligomer or polymer of the monomeric form.

4. Test carrier according to claim 2, characterized in that the second binding partner is increased in size by chemical bonding with a binding partner which is inert as regards the biological affinity binding reaction.

5. Test carrier according to any one of the preceding claims, characterized in that the first binding partner comprises streptavidine and the second binding partner comprises biotin.

6. Test carrier according to claim 1, characterized in that a colour detection layer (20) is disposed in the fluid transport path downstream from the fixing layer (18) and an optical blocking layer (19) is disposed between the fixing layer (18) and the colour detection layer (20).

7. Test carrier according to any one of the preceding claims, characterized in that the thickness of the fixing layer is less than 0.5 mm, in particular less than 0.25 mm.

8. Test carrier according to any one of the preceding claims, characterized in that the fixing layer is associated with a flow adjusting element (5, 22) by means of which the time at which the sample fluid flows through the fixing layer can be controlled.

9. Test carrier according to claim 8, characterized in that the fixing layer (18) is fixed to the test carrier (10) in such a way that, when the test carrier (10) is in its initial state, it is not in fluid contact with a fluid feed layer (16) which is located immediately upstream in the fluid transport path, but can be brought into fluid contact with the fluid feed layer (16).

10. Test carrier according to claim 9 in conjunction with claim 6, characterized in that the fixing layer (18), the optical barrier layer (19) and the colour detection layer (20) are disposed in this sequence like a flap above the fluid feed layer.

11. Test carrier according to any one of the preceding claims, characterized in that the fixing layer (18) consists of polyvinyl difluoride, polyamide, polystyrene or nitrocellulose.

## Revendications

1. Support de test pour la détermination analytique d'un composant dans un liquide de prélèvement à l'aide d'une réaction spécifique de liaison de deux partenaires de liaison bioaffines, muni d'une multitude de couches de test qui sont disposées de façon à être imprégnées les unes après les autres par le liquide et à constituer un parcours de liquide (7), une des couches de test étant une couche de fixation (4, 18) qui présente une matrice porteuse poreuse sur laquelle est fixé le premier des deux partenaires de liaison, la couche de fixation (4, 18) étant disposée sur le support de test (1, 10) de façon à être traversée perpendiculairement à sa surface par le liquide qui contient le second partenaire de liaison, et au moins une couche absorbante (3, 19, 20) étant prévue dans le parcours de liquide (7), derrière la couche de fixation (4, 18), caractérisé en ce que la matrice porteuse de la couche de fixation (4, 18) est une couche de plastique microporeuse avec une grosseur de pores P d'au moins 0,01 $\mu$m et en ce que la grosseur D du second partenaire de liaison ainsi que la grosseur de pores P de la couche de plastique sont adaptées l'une à l'autre de telle sorte que la grosseur de pores P est au moins deux et au maximum dix fois aussi importante que la grosseur D du second partenaire de liaison.

2. Support de test selon la revendication 1, caractérisé en ce que le second partenaire de liaison est agrandi par liaison chimique à un poids moléculaire supérieur à celui auquel correspond sa forme monomère.

3. Support de test selon la revendication 2, caractérisé en ce que le second partenaire de liaison est un oligomère ou un polymère de la forme monomère.

4. Support de test selon la revendication 2, caractérisé en ce que le second partenaire de liaison est agrandi par liaison chimique avec un partenaire de liaison inerte par rapport à la réaction de liaison bioaffine.

5. Support de test selon la revendication 2, caractérisé en ce que le premier partenaire de liaison contient de la streptavidine et le second partenaire de liaison de la biotine.

6. Support de test selon la revendication 1, caractérisé en ce qu'une couche d'indication colorée (20) est disposée dans le parcours de liquide, derrière la couche de fixation (18), et une couche optique d'arrêt (19) entre la couche de fixation (18) et la couche d'indication colorée (20).

7. Support de test selon l'une des revendications précédentes, caractérisé en ce que l'épaisseur de la couche de fixation est inférieure à 0,5 mm et, en particulier, inférieure à 0,25 mm.

8. Support de test selon l'une des revendications précédentes, caractérisé en ce qu'un élément de contrôle de l'écoulement (5, 22) à travers lequel peut être commandé le moment de passage du liquide de prélèvement par la couche de fixation est affecté à la couche de fixation.

9. Support de test selon la revendication 8, caractérisé en ce que la couche de fixation (18) est fixée au support de test (10) de façon à ne pas être en contact fluidique avec une couche d'amenée du liquide préalablement logée dans le parcours de liquide (16), lorsque le support de test (10) est à l'état de sortie mais à pouvoir être mise en contact fluidique avec la couche d'amenée du liquide (16).

10. Support de test selon la revendication 9 en liaison avec la revendication 6, caractérisé en ce que la couche de fixation (18), la couche optique d'arrêt, (19) et la couche d'indication colorée (20) sont disposées dans cet ordre, comme un clapet, au-dessus de la couche d'amenée du liquide.

11. Support de test selon l'une des revendications précédentes, caractérisé en ce que la couche de fixation (18) se compose de difluorure de polyvinyle, de polyamide, de polystyrol ou de nitrocellulose.

Fig. 1

Fig. 2